# NEW EUROPEAN PATENT SPECIFICATION

(11) **EP 0 401 189 B2**
(45) Date of publication and mention of the opposition decision: **26.06.1996**
(45) Mention of the grant of the patent: 28.07.1993
(21) Application number: 90850217.2
(22) Date of filing: 29.05.1990
(51) Int. Cl.: A61F 13/46, A61F 13/52, A61L 15/60

(54) **Absorbent article comprising at least two superabsorbents**
Absorptionsartikel, enthaltend wenigstens zwei Superabsorbers
Article absorbant comprenant au moins deux superabsorbants

(30) Priority: 31.05.1989 SE 8901964
(43) Date of publication of application: 05.12.1990
(60) Divisional application: 93100289.3
(73) Proprietor: Mölnlycke AB, 405 03 Göteborg (SE)
(72) Inventor: Roos, Anders, S-412 57 Göteborg (SE); Quist, Magnus, S-448 00 Floda (SE); Hermansson, Jonas, S-412 61 Göteborg (SE)
(74) Representative: Hyltner, Jan-Olof

(56) References cited:
- GB-A- 2 145 661
- US-A- 4 212 302
- US-A- 4 333 464
- US-A- 4 338 371
- US-A- 4 578 068
- US-A- 4 578 068
- US-A- 4 834 735

## Description

The present invention relates to an absorbent article, according to the préamble of claim 1.

Absorbent bodies or pads for disposable articles of the aforesaid kind, i.e. articles intended for one time use only, have, for many years, normally been produced essentially from absorbent fibre material. The fibre material used has most often been cellulose fluff material. In recent years, absorbent materials having a much greater absorption capacity than fluff have been developed and it has become progressively more general to incorporate these highly absorbent materials, so-called superabsorbents, in sanitary articles. Since the superabsorbents are able to absorb liquid in quantities many times greater than their own weight, the use of such materials in sanitary articles enables conciderable savings to be made in other kinds of absorption material, such as cellulose fluff. This is particularly desirable, since the articles can thereby be made thinner and therewith worn more discretely, which is a particularly important advantage in the case of adult incontinence.

It has been found, however, that although superabsorbents are advantageous with regard to their large liquid-absorbing capacities, they are also encumbered with certain drawbacks. For instance, when a super-absorbent absorbs liquid the phenomenom known as gel-blocking occurs. As a result of swelling, entrance to the non-utilized absorption capacity of the superabsorbent, which comprises a polymergel, is denied to the liquid and consequently liquid which is not absorbed, due to gel-blocking, will run in an uncontrolled fashion along the surface of the absorbent pad and ultimately leak from the pad and cause discomfort to the wearer. It is also found that different superabsorbents will absorb liquid at different rates. The absorption rate of some superabsorbents is too slow for the absorbents to have a practical use in sanitary products. Other superabsorbents have the drawback known as re-wetting, i.e. the gel is unable to retain the liquid contained therein when subjected to pressure and, consequently, liquid flows back to the surface material of the article, causing the surface material to become moist or wet and therewith subjecting the wearer to a great deal of discomfort.

Although several endeavours have been made to solve this problem, none of these endeavours have hitherto provided satisfactory results. A proposed solution to gel-blocking is disclosed, inter alia, in GB 2 145 661, according to which superabsorbent material is disposed in discrete layers with liquid-dispersing layers between the superabsorbent layers. The superabsorbents mentioned in this publication, however, are of mutually the same type and consequently the wearer is at the mercy of the properties of the superabsorbents with regard to the absorption properties of the sanitary article and its function. US 4,578,068 describes an absorbent pad which comprises a plurality of layers of fibre material with superabsorbents disposed between the layers. According to this publication, the superabsorbents may also be of mutually the same kind or may corn prise a mixture of mutually different superabsorbents, although the properties of these superabsorbents is not disclosed.

The object of the present invention is to provide an absorbent article which comprises an absorbent pad having improved absorption properties with respect to earlier known absorbent pads, said absorbent pad combining the essential properties of an absorbent pad such as absorption rate and extremely good liquid-retention properties.

In accordance with the invention, this is achieved with an absorbent article comprising an absorbent pad, which exhibits the characterizing features of claim 1.

It has surprisingly been found that the ability of the absorbent pad to absorb liquid can be substantially improved by incorporating in the pad two superabsorbents which differ from one another with respect to absorption rate and to re-wetting tendency respectively. It is highly important to the invention that the different superabsorbents are disposed in separate layers or in separate regions of the absorbent pad or body. It is found that the absorbent pad will have far better absorption properties when the superabsorbents are held separated from one another than if said absorbents are mixed together. Similarly, the incorporation of two superabsorbents will provide considerably better absorption than when only one of the superabsorbents is incorporated in the absorbent pad and the other excluded therefrom. The mutually relative location of the superabsorbents in the absorbent pad is of great significance. The superabsorbent with exhibits a high absorption rate should be placed beneath the superabsorbent which exhibits low re-wetting tendencies, as seen from the side surface facing towards the wearer, i.e. the liquid-receiving side-surface, for obtaining better absorption properties.

An absorbent article constructed in accordance with the invention will now be described in more detail with reference to an exemplifying embodiment illustrated in the accompanying drawings.

Figure 1 is a view from above of an inventive absorbent article with the side of the article which faces towards the wearer in use facing towards the viewer.

Figure 2 is a cross-sectional view of the article shown in Figure 1, taken on the line II-II in said Figure.

Figure 3 is a view from above of another embodiment of an inventive absorbent article, with the side which faces the wearer in use facing towards the viewer.

Figure 4 is a cross-sectional view of the absorbent article shown in Figure 3, taken on the line IV-IV in said Figure.

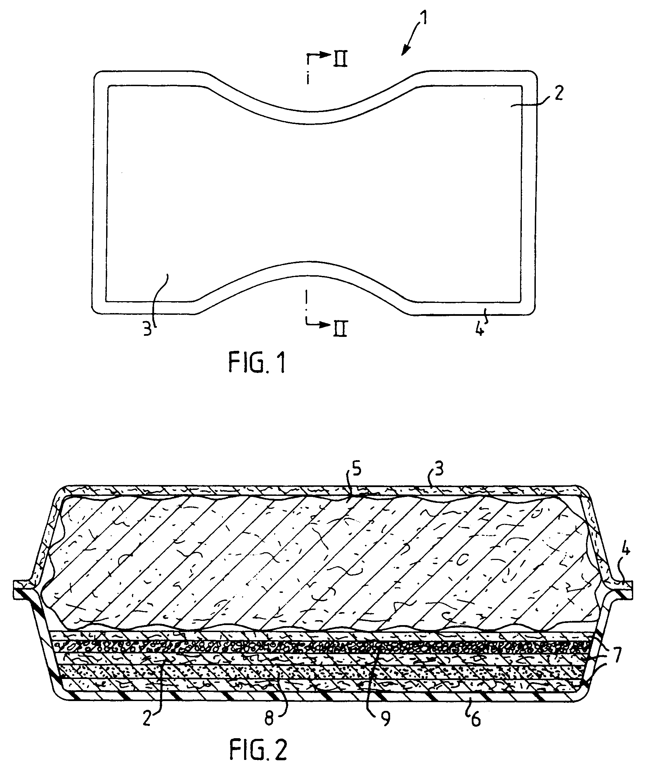

Figures 1 and 2 illustrate an absorbent article 1, which is assumed to be a disposable diaper and which comprises a liquid-permeable sheet 3, preferably made of nonwoven fibre fabric. The liquid-permeable sheet 3 is joined at the edge-parts 4 of the diaper to a liquid-impermeable sheet 6 in a conventional manner, e.g. with the aid of an adhesive binder. The liquid-impermeable sheet 6, which comprises polyethylene for instance, functions to prevent liquid from coming into contact with the wearer's clothes or to prevent liquid absorbed in the diaper from leaking therefrom.

Located beneath the liquid-permeable sheet 3 is a body of fibre wadding 5. The fibre wadding has a loose structure, is elastically resilient, and is shape-stable in both a dry and a wet state. The wadding comprises natural or synthetic fibres, such as polyester, polypropylene or polyethylene fibres, or mixtures thereof. So-called bicomponent fibres are suitable materials in this respect, by which is meant fibres composed of two types of polymer, for instance polypropylene/polyethylene, polyester/polyester or polyester/polyethylene. The fibre wadding 5 may also either be bound or non-bound with regard to its structure. Heat and adhesive bonding agents are examples of the means by which a fibre wadding structure can be bound. The adhesive binding agent may be a latex binder, for instance.

An absorbent body, generally referenced 2, is placed between the fibre wadding 5 and the liquid-impermeable sheet 6 distal from the wearer. The absorbent pad 2 comprises a plurality of distance-maintaining dispersion layers 7 and superabsorbent materials 8, 9 disposed between said layers. The absorbent pad of the Figure 2 embodiment includes three distance-maintaining dispersion layers 7, although this number may be greater than three. The layers 7 may, for instance, comprise one or more layers of tissue, although other materials, such as nonwoven or compressed cellulose fluff, may be used. It is important to the invention that the superabsorbent layers 8, 9 are held mutually separated and that the layer 9 is positioned closer to the wearer of the article than the layer 8. Thus, in the case of the Figure 2 embodiment, a tissue layer 7 is placed immediately beneath the wadding 5 and is followed sequentially by a superabsorbent layer 9, a tissue layer 7, a superabsorbent layer 8 and a tissue layer 7. The superabsorbents in the layers 8, 9 are preferably in powder form, although other forms are conceivable, such as a fibre form for instance. The superabsorbents in the layer 9 comprise a superabsorbent which exhibits very good liquid-retention properties, high surface-dryness and low re-wetting tendency. Such a superabsorbent is also characterized by a high gel strength. The superabsorbents in the layer 8, on the other hand, comprise a superabsorbent which exhibits a very high absorption rate.

The use of two superabsorbents having such radically different absorption properties as described above gives a highly surprising an unexpected result with regard to the absorption rate and re-wetting tendency of the absorbent pad as a whole. This will become more evident from the following example.

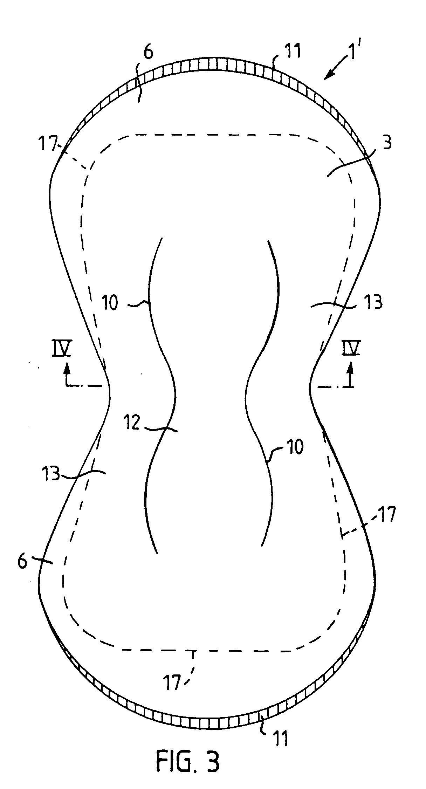

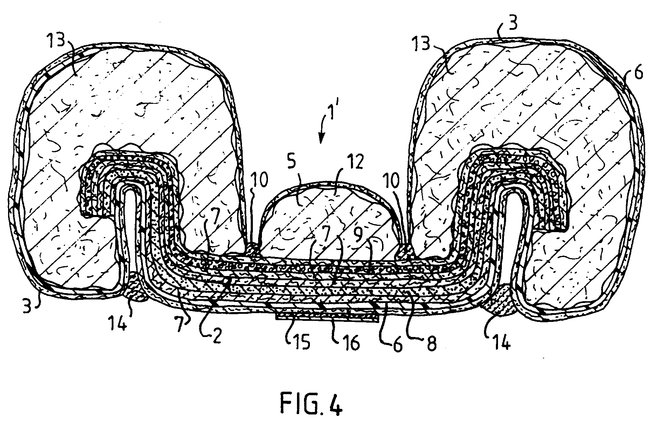

The absorbent article 1' illustrated in Figures 3 and 4 is assumed to be an incontinence guard. With regard to the choice of materials and positioning of the fibre wadding 5, absorbent pad 2 and liquid-impermeable sheet 6, the construction of the incontinence guard 1' is identical to that of the diaper 1. Like reference signs in Figures 1, 2 and 3, 4 identify identical elements.

The incontinence guard 1' differs from the diaper 1 in a number of respects, however.

Similar to the diaper 1 of Figures 1 and 2, the incontinence guard 1' comprises a liquid-permeable sheet 3, e.g. of nonwoven material, which is located nearest the wearer in use. This sheet, however, surrounds the incontinence guard around the whole of its periphery.

The incontinence guard 1' is divided by two longitudinally extending, curved fold lines 10, e.g. formed by weld seams or by adhesive bonds, into a central part 12 and two edge-parts 13. The intention of the fold lines 10 is to fold-up the edge-parts 13 along said lines and to fixate said upwardly folded edge-parts in a permanent upstanding position with the aid of joining means 14, e.g. a binding agent or a welded seam, such as to obtain a three-dimensional basin-like absorbent article in which the edge-parts 13 function as sealing embankments in the lateral direction of the article.

The parts 12, 13 are relatively elongated, i.e. their extension in the longitudinal direction of the article is greater than the extension of the article in its transverse direction. The end-parts of the incontinence guard are provided with joins 11, which may, for instance, have the form of welded joins or adhesive joins.

When the fold lines 10 comprise welded joins or seams, such joins can be produced by appropriate, conventional welding techniques, e.g. ultrasonic welding, impulse welding or high frequency welding. The fold lines 10 may also have a linear or arcuate shape, as an alternative to the curve shape shown in Figure 3.

The fold lines 10 may either be continuous or discontinuous. For instance, the fold lines may be formed by a broken curve of punctiform welds.

Located immediately beneath the absorbent pad 2, as seen from the wearer, is a liquid-impermeable sheet 6, which extends slightly up along the edge-parts and end-parts of the incontinence guard 1' and somewhat in over the fibre wadding 5 on the side surface of the incontinence guard 1' facing towards the wearer in use. The extension of the liquid-impermeable layer 6 is shown with a broken line 17 in Figure 3.

Positioned on the underside of the incontinence guard is an adhesive layer 15, by means of which the guard can be removably attached to the underclothes of the wearer. Prior to using the guard 1', the adhesive layer 15 is protected by a protective layer 16, which is preferably siliconized or treated with a release agent in some other way, so that when the guard 1' is used, the protective material can be readily removed and therewith render the adhesive layer 15 active.

The absorption properties of an inventive absorbent article with respect to re-wetting tendencies will now be illustrated in the following example.

### Example 1

The absorption rate of six superabsorbents was measured in accordance with the Vortex-analysis method. The six superabsorbents tested were all available commercially and sold under the trade names Aqualic CA W-4, Waterlock J 550, Aridall 1125, Aridall 1092, Sanwet IM 2200 D and Sanyo PA 200G.

A beaker containing 50 ml NaCl (aq) in a quantity of 9 g/liter was placed on a magnetic stirrer. The stirrer was set to rotate at a speed of 600 ± 20 rpm. When the resultant Vortex has stabilized, 2.0 g of superabsorbent were poured into the beaker, externally of the Vortex. A measurement was taken of the time that lapsed from the moment of adding the superabsorbent to the beaker to the moment that the Vortex disappeared and a completely smooth surface was obtained. The procedure was repeated the requisite number of times.

The following results were obtained:

**Table 1**

| Superabsorbent | Vortex time (s) |
|---|---|
| Aqualic CA W-4 | 21 |
| Aridall 1092 | 5 |
| Aridall 1125 | 7 |
| Sanwet IM 2200 D | 67 |
| Sanyo PA 200 G | 44 |
| Waterlock J 550 | 2 |

Waterlock J 550, Aridall 1092 and Aridall 1125 were found to have much shorter absorption times than the other superabsorbents tested. Waterlock J 550 is used in Examples 3-5 as an example of a superabsorbent which possesses a very high absorption rate.

### Example 2

Measurements were made of the gel strength of the same superabsorbents as those whose absorption rates were measured in Example 1.

The gel strength measurements were taken with the aid of a Carri-Med C.S. rheometer. The measurements were taken with the following parameters:
- Measuring program: Oscillation
- Measuring system: Plate-plate (serrated)
- Plate radius: 2 cm
- Plate distance: 2 mm
- Oscillation frequency: 1.0 Hz
- Torque: 1.00 Nm
- Temperature: 20°C
30 ml of 0.9% NaCl-solution were poured into a 100 ml beaker. 1.00 g of superabsorbent was added to the solution, while stirring with a magnetic stirrer. Stirring was stopped when the superabsorbent had absorbed the total quantity of liquid present, whereafter the resultant gel was allowed to stand for one hour. The gel was then mixed and a sample of 5 ml was taken. The sample was placed centrally on the bottom plate, whereafter the measuring process was commenced.

Table 2 shows the values obtained with an energy input of 3 mJ.

**Table 2**

| Superabsorbent | Gel strength (kPa) |
|---|---|
| Aqualic CA W-4 | 3.2 |
| Aridall 1092 | 0.2 |
| Aridall 1125 | 0.3 |
| Sanwet IM 2200 D | 2.9 |
| Sanyo PA 200 G | 2.9 |
| Waterlock J 550 | 1.4 |

Aqualic CA W-4, Sanwet IM 2200 D and Sanyo PA 200 G exhibited high gel strength, whereas the remaining superabsorbents exhibited moderate or low gel strength.

A relationship between high gel strength and low rewetting tendency, i.e. high liquid-retention, can be shown even though no clear relationship exists. Without intending to provide a complete explanation of this relationship, it can be mentioned that a strongly cross-linked, dense and hard gel is more difficult to compress than a gel having the opposite properties, and consequently it is more difficult to press absorbed liquid from a gel of the firstmentioned kind than from the lastmentioned kind. Furthermore, the greater suction force of a strongly cross-linked superabsorbent contributes to the low re-wetting tendency of said absorbent. Higher degrees of cross-linking result in finer capillary systems, which in turn result in higher suction forces. Low re-wetting tendencies can also be obtained by subjecting the gel to different types of surface treatment.

### Example 3

The superabsorbents recited in Examples 1 and 2 were tested for re-wetting.

5 g of a superabsorbent were spread uniformly in the form of a layer in the bottom, rectangular absorbent pad of a diaper sold under the trade name Libero Super. 100 ml of an 0.9% NaCl-solution were poured through a pipe onto the wetting-location of the diaper. The time taken for the diaper to absorb the liquid was measured and recorded. After 10 minutes, filter papers, having a diameter of 80 mm, were placed over the wetting-location and subjected to a load of 1.5 kg for 15 seconds. The filter papers were weighed before and after applying said load and re-wetting was recorded.

The method provides a measurement of the ability of the superabsorbent to absorb liquid and to retain said liquid when subjected to load, when the superabsorbent is incorporated in an absorbent article.

The results are shown in Table 3.

**Table 3**

| Superabsorbent | Re-wetting (ml) |
|---|---|
| Aqualic CA W-4 | 1 |
| Aridall 1092 | 21 |
| Aridall 1125 | 10 |
| Sanwet IM 2200 D | 6 |
| Sanyo PA 200 G | 1 |
| Waterlock J 550 | 12 |

Aqualic CA W-4 and Sanyo PA 200 G were found to have by far the lowest re-wetting tendency of the superabsorbents tested, this being in agreement with the gel strength measurements of Example 2. Aqualic CA W-4 was used in the following examples as an example of a surface-dry superabsorbent having very high liquid-retention properties.

### Example 4

Absorption rate, liquid leakage in sloping planes and re-wetting were measured in respect of an absorbent pad or body which incorporated two mutually separate superabsorbent layers. The results are compared with the results obtained in respect of a reference body which included an absorbent pad comprising a layer of a mixture of the superabsorbents.

The firstmentioned absorbent pad comprised an upper tissue-laminate in which 2.5 g Aqualic CA W-4 were placed in powder form, and a bottom tissue-laminate which contained 2.5 g Waterlock J 550 relative to the liquid-receiving surface material.

The last mentioned absorbent body included a tissue laminate which contained a mixture of varying quantities Aqualic CA W-4 and Waterlock J 550.

All reference bodies included a nonwoven material , manufactured by Shiseida, and two pieces of fibre wadding, sold under the trade mark Dacron, placed on top of the absorbent pad.

### Absorption rate

The reference bodies were laid flat and 100 ml 0.9 % NaCl (aq) were poured onto respective bodies over a period of 7 seconds and the time taken for the body to absorb the liquid was measured (visual observation). The results are presented in Table 4 below.

### Liquid leakage in sloping planes

The reference body was placed on an inclined surface, the angle of said surface to the horizontal being 30%. 100 ml of 0.9% NacL (aq) were poured onto the upper end of the body over a period of 7 seconds. The quantity of non-absorbed liquid was collected at the lower end of the body and weighed. The results are presented in Table 4 below.

### Re-wetting

A plate weighing 3.5 kg was laid on top of the reference body. 50 ml of 0.9% Nacl (aq) were poured onto the reference body and the body was left to absorb the liquid for a period of 3 minutes.

Filter papers weighing 5 g and having a diameter of 50 mm were placed on the wetting location and loaded with a pressure of 8 kg/dm² for 1 minute.

The filter papers were then weighed and the increase in weight of said papers taken as a re-wetting measurements. The results are presented in Table 4 below.

Aqualic CA W-4 is referenced AL and Waterlock J 550 is referenced WL.

**Table 4**

| Type of abs.body | Quantity | | Absorption-time (s) | Leakage in sloping plane (g) | Re-wetting (g) |
|---|---|---|---|---|---|
| | AL (g) | WL (g) | | | |
| Mixing | 4 | 1 | 5.9 | 11.0 | 3.2 |
| Mixing | 3 | 2 | 3.0 | 9.5 | 3.1 |
| Mixing | 2 | 3 | 2.2 | 9.7 | 3.6 |
| Mixing Separate | 1 | 4 | 1.6 | 8.7 | 3.5 |
| layers | 2.5 | 2.5 | 2.1 | 0 | 0.6 |

The reference bodies which comprised a relatively large proportion of rapidly-absorbing superabsorbent, in the example at least 2/5, were found to have a short absorption time, irrespective of whether the superabsorbents were mixed or separated, although the reference body in which the superabsorbents were mutually separated was found to have considerably better values with regard to liquid leakage in a sloping plane and re-wetting than the reference bodies which included a layer comprising a mixture of the same superabsorbents.

### Example 5

Absorption rate, liquid leakage in a sloping plane and re-wetting were measured in respect of a series of reference bodies which contained various different superabsorbents placed in mutually separate layers.

The analyses were carried out in the same manner as that recited in Example 4. In all cases, the absorbent pad comprised an upper tissue-laminate containing 2.5 g superabsorbent, and a lower tissue-laminate containing 2.5 g of a superabsorbent different to the superabsorbent in the upper laminate.

The values obtained are presented in Table 5 below.

**Table 5**

| Absorbent pad | | Absorption time (s) | Leakage in sloping plane (g) | Re-wetting (g) |
|---|---|---|---|---|
| U-layer | L-layer | | | |
| AL | S | 9.6 | 14.6 | 1.1 |
| AL | A10 | 4.8 | 15.0 | 2.5 |
| AL | A11 | 7.2 | 15.8 | 1.7 |
| AL | SW | 13.8 | 21.0 | 1.0 |
| AL | WL | 2.1 | 0 | 0.6 |
| WL | S | 2.6 | 11.7 | 2.7 |
| WL | A10 | 2.3 | 8.4 | 3.4 |
| WL | A11 | 2.9 | 12.4 | 4.1 |
| WL | SW | 2.7 | 10.7 | 3.1 |
| WL | AL | 2.0 | 7.3 | 2.0 |
| U-layer = Upper layer L-layer = Lower layer AL = Aqualic CA W-4 WL = Waterlock J 550 S = Sanyo PA 200 G A 10 = Aridall 1092 A 11 = Aridall 1125 SW = Sanwet IM 2200 D | | | | |

The results show that the absorption time is much shorter in those instances when the superabsorbent which in Example 1 exhibited the fastest absorption rate, Waterlock J 550, is present. The lowest re-wetting tendency is obtained with those absorbent pads which contain Aqualic CA W-4, which in Example 2 was found to have the highest gel strength and, in Example 3, a very low re-wetting tendency.

The combination of a superabsorbent of low re-wetting tendency in an upper layer and a superabsorbent of high absorption rate in a lower layer gives by far the best result with respect to liquid-leakage in a sloping plane. The re-wetting tendency is also lowest with this combination.

### Example 6

Absorption rate, liquid-leakage in a sloping plane and re-wetting were measured in respect of a series of reference bodies in which the superabsorbents were incorporated in respective reference bodies in varying quantities, said measurements being taken in the same manner as that recited in Examples 4 and 5. The total amount of superabsorbent in each body was 5 g. The absorbent pads comprised an upper tissue-laminate of Aqualic CA W-4, and a lower tissue-laminate of Waterlock J 550. In some instances, one of the superabsorbents was omitted, i.e. the absorbent pad in question comprised solely one single-laminate containing one single superabsorbent.

The average measurement values are presented in Table 6 below. Aqualic CA W-4 is abbreviated to AL in Table 6 and Waterlock J 550 is abbreviated to WL.

**Table 6**

| Amount of superabsorbent (g) | | Absorption time (s) | Leakage in sloping plane (g) | Re-wetting (g) |
|---|---|---|---|---|
| AL | WL | | | |
| 5 | 0 | 13.98 | 6.0 | 0.88 |
| 4 | 1 | 3.09 | 2.1 | 0.58 |
| 3 | 2 | 2.29 | 0 | 0.47 |
| 2.5 | 2.5 | 2.14 | 0 | 0.46 |
| 2 | 3 | 2.80 | 0.5 | 0.64 |
| 1 | 4 | 1.25 | 0 | 0.81 |
| 0 | 5 | 0.97 | 2.3 | 3.36 |

It will be seen from Table 6 that the absorption time is much longer in those cases when the rapidly-absorbing superabsorbent is not present than in other cases. A higher re-wetting value is obtained for the absorbent pad which contains solely Waterlock J 550 than for the other absorbent pads. The values obtained in respect of liquid-leakage in a sloping plane show that the presence of both types of superabsorbent provides the best results.

It will be understood that the invention is not restricted to the examples nor to the embodiments illustrated in the drawings, and that several modifications are possible within the scope of the following claims.

Furthermore, as will be understood, an absorbent pad can be constructed from more than two mutually different superabsorbents having substantially differing absorption properties.

Instead of placing superabsorbents between at least two layers of material, i.e. in a laminate form, the superabsorbent may alternatively be placed on a material layer or mixed into said material layer, which in this latter case will preferably comprise a layer of fluff.

The invention has been described in the aforegoing exemplifying embodiments with reference to a diaper or an incontinence guard, although it will be understood that the invention can also be applied to other absorbent articles, such as sanitary towels or panty-protectors for example.

## Claims

1. An absorbent article intended for one-time-use only, such as a diaper, an incontinence guard, a sanitary towel or the like, comprising a liquid-permeable sheet (3), which faces the wearer in use, an absorbent pad (2), and a liquid-impermeable sheet (6) which is distal from the wearer in use, **characterized** in that the absorbent pad includes at least two mutually different superabsorbents, which differ from one another with respect to absorption rate and to liquid-retention ability under pressure, respectively; in that the mutually different superabsorbents are disposed in at least two separate layers (8,9) which are mutually separated by means of one or more distance-maintaining dispersion layers (7); and in that the superabsorbent layer (8), which includes a superabsorbent which exhibits a high absorption rate, is located beneath the superabsorbent layer (9), which includes a superabsorbent which has a high liquid-retention ability when subjected to pressure.

2. An article according to Claim 1, **characterized** in that the superabsorbent layers (8,9) comprise superabsorbents disposed between at least two material layers (7).

3. An article according to Claim 1, **characterized** in that the superabsorbent layers (8,9) comprise superabsorbents placed on a material layer.

4. An article according to Claim 1 or 2, **characterized** in that at least one of the superabsorbent layers (8,9) comprises a superabsorbent mixed into a material layer.

5. An article according to Claim 2,3 or 4, **characterized** in that the material layers (7) are formed from tissue, nonwoven material, fluff, cotton wool or perforated plastic film.

6. An article according to any of the preceding Claims, **characterized** in that fiber wadding (5) is placed between the liquid-permeable sheet (3) and the absorbent pad (2).

## Patentansprüche

1. Absorbierender Artikel zur Einmalverwendung, wie zum Beispiel eine Windel, ein Inkontinenz-Schutz, eine Damenbinde oder dergleichen, mit einer flüssigkeitsdurchlässigen Schicht (3), die beim Gebrauch auf die Trägerperson zuweist, einem absorbierenden Kissen (2) und einer flüssigkeitsundurchlässigen Schicht (6), die beim Gebrauch von der Trägerperson abgewandt ist, dadurch **gekennzeichnet,**
daß das absorbierende Kissen wenigstens zwei untereinander unterschiedliche, hoch absorbierende Materialien umfaßt, die sich voneinander bereits in bezug auf die Absorptionsgeschwindigkeit und die Fähigkeit unterscheiden, Flüssigkeit unter Druck festzuhalten,
daß die untereinander unterschiedlichen, hochabsorbierenden Materialien in wenigstens zwei getrennten Schichten (8, 9) angeordnet sind, die voneinander mit Hilfe von einer oder mehreren abstandshaltenden Dispersionsschichten (7) getrennt sind, und
daß die hochabsorbierende Schicht (8) ein hochabsorbierendes Material enthält, das eine hohe Absorptionsgeschwindigkeit aufweist, unterhalb der hochabsorbierenden Schicht (9) liegt, die ein hochabsorbierendes Material enthält, das eine hohe Fähigkeit hat, Flüssigkeit dann festzuhalten, wenn es einem Druck ausgesetzt wird.

2. Artikel nach Anspruche 1, dadurch gekennzeichnet, daß die hochabsorbierenden Schichten (8, 9) hochabsorbierende Materialien enthalten, die zwischen wenigstens zwei Materialschichten (7) angeordnet sind.

3. Artikel nach Anspruch 1, dadurch gekennzeichnet, daß die hochabsorbierenden Schichten (8, 9) hochabsorbierende Materialien enthalten, die auf einer Materiallage angeordnet sind.

4. Artikel nach einem der Ansprüche 1 oder 2, dadurch gekennzeichnet, daß wenigstens eine der hochabsorbierenden Schichten (8, 9) ein hochabsorbierendes Material enthält, das in eine Materiallage eingemischt ist.

5. Artikel nach einem der Ansprüche 2, 3 oder 4, dadurch gekennzeichnet, daß die Materialschichten (7) aus Gewebe, nichtgewebtem Material, Flocken, Rohbaumwolle oder einem perforierten Kunststoffilm hergestellt sind.

6. Artikel nach einem der vorangegangenen Ansprüche, dadurch gekennzeichnet, daß zwischen der flüssigkeitsdurchlässigen Schicht (3) und dem absorbierenden Kissen (2) eine Faserwattierung (5) angeordnet ist.

## Revendications

1. Article absorbant à usage unique, tel une couche-culotte, une protection pour incontinent, une serviette hygiénique ou autre article similaire, comprenant une feuille (3) perméable aux liquides se trouvant, en utilisation, contre l'utilisateur, un coussin absorbant (2) et une feuille (6) imperméable aux liquides se trouvant, en utilisation, loin de l'utilisateur, caractérisé en ce que le coussin absorbant inclut au moins deux superabsorbants différents l'un de l'autre, qui diffèrent l'un de l'autre en ce qui concerne, respectivement, la vitesse d'absorption et la capacité de rétention de liquide lorsqu'ils sont soumis à la pression, en ce que les superabsorbants différents l'un de l'autre sont disposés en au moins deux couches séparées (8, 9), qui sont séparées l'une de l'autre au moyen d'une ou plusieurs couches de dispersion (7) maintenant l'écartement, et en ce que la couche superabsorbante (8), qui contient un superabsorbant présentant une vitesse d'absorption élevée, est située en-dessous de la couche superabsorbante (9) qui contient un superabsorbant présentant une capacité de rétention de liquide élevée lorsqu'il est soumis à la pression.

2. Article selon la revendication 1, caractérisé en ce que les couches superabsorbantes (8, 9) contiennent des superabsorbants disposés entre au moins deux couches de matériau (7).

3. Article selon la revendication 1, caractérisé en ce que les couches superabsorbantes (8, 9) contiennent des superabsorbants placés sur une couche de matériau.

4. Article selon la revendication 1 ou 2, caractérisé en ce que l'une au moins des couches superabsorbantes (8, 9) contient un superabsorbant mélangé dans une couche de matériau.

5. Article selon la revendication 2, 3 ou 4, caractérisé en ce que les couches de matériau (7) sont faites de tissu de papier, de matériau non tissé, de fluff, de laine de coton ou d'un film plastique perforé.

6. Article selon l'une quelconque des revendications précédentes, caractérisé en ce qu'un rembourrage de fibres (5) est placé entre la feuille (3) perméable au liquide et le coussin absorbant (2).
